# EUROPÄISCHE PATENTSCHRIFT

(11) **EP 0 370 998 B1**
(45) Veröffentlichungstag und Bekanntmachung des Hinweises auf die Patenterteilung: **02.06.1993**
(21) Anmeldenummer: 89890301.8
(22) Anmeldetag: 20.11.1989
(51) Int. Cl.: A61C 1/18

(54) **Winkelstückkupplung**
Elbow coupling
Raccord de pièce à main dentaire

(30) Priorität: 21.11.1988 AT 2854/88
(43) Veröffentlichungstag der Anmeldung: 30.05.1990
(73) Patentinhaber: DENTALWERK BÜRMOOS GESELLSCHAFT M.B.H., 5111 Bürmoos (AT)
(72) Erfinder: Malata, Peter jun., Dipl.-Ing., A-5111 Bürmoos (AT)
(74) Vertreter: Barger, Werner, Dipl.-Ing.

(56) Entgegenhaltungen:
- EP-A- 0 185 290
- DE-U- 8 322 850
- FR-A- 2 601 870
- US-A- 4 600 384

## Beschreibung

Die Erfindung betrifft eine Verbindung zahnärztlicher Winkelstücke mit zahnärztlichen Handstücken, wobei nahe des Werkzeughalters eine Austrittsstelle für einen Lichtleiter vorgesehen ist, um die Arbeitsstelle im Mund des Patienten zu beleuchten und wobei die entsprechende Lichtquelle entweder im Winkelstück oder im Handstück vorgesehen ist.

Es ist eine ganze Reihe von zahnärztlichen Winkelstücken mit solchen Lichtleitern bekannt, doch haben sich in der Praxis zwei Arten durchgesetzt.

Beim ersten etablierten System sitzt die Lichtquelle im Handstück und ihr gegenüber im Winkelstück befindet sich das lichtaufnehmende Ende des Lichtleiters (siehe EP-A-185290). Um dieses Gegenüberliegen zu sichern, ist in der ansonsten rotationssymmetrischen Kupplung eine Nase vorgesehen, die die richtige Winkellage zwischen Winkelstück und Handstück sicherstellt.

Beim zweiten häufig vorkommenden System befindet sich die Lichtquelle im Winkelstück (siehe US-A-4600384) und wird mittels konzentrischer, kreisförmiger Schleifringe an der Stirnfläche der Kupplung unabhängig von der Winkellage zwischen Winkelstück und Handstück, vom Handstück aus mit Strom versorgt.

Die Existenz dieser beiden Beleuchtungssysteme bringt sowohl für die Hersteller der Winkelstücke als auch für die Zahnärzte eine ganze Reihe von Unannehmlichkeiten mit sich. Die Handstück werden üblicherweise von den Firmen gefertigt, die auch die Patientensessel für Zahnarztpraxen herstellen. Die Hersteller der Winkelstücke fertigen im allgemeinen keine Zahnarztsessel und die Hersteller der Sessel wiederum im allgemeinen keine Winkelstücke. Die Winkelstückhersteller müssen somit bei der Herstellung und bei der Lagerhaltung die beiden verschiedenen Systeme beachten und sind gezwungen, ihre Lager auszuweiten. Dies wirkt sich selbstverständlich auf die Kosten und auf die Lieferzeit nachteilig aus.

Die Benutzer wiederum sind in der Auswahl der Winkelstücke durch das Handstücksystem, das sie gewählt haben, gebunden und müssen, wenn an ihrem Arbeitsplatz Anschlüsse für beide Arten von Handstücken vorgesehen sind, streng darauf achten, die entsprechenden Handstücke nicht zu verwechseln bzw. oft beim Wechseln des Winkelstückes auch das Handstück zu wechseln. Dies wiederum ist lästig und bringt Probleme bei der Sterilisation der Instrumente nach der Behandlung eines Patienten mit sich.

Es ist das Ziel der Erfindung, diese Nachteile zu vermeiden und eine Verbindung der eingangs erwähnten Art zu schaffen, die mit möglichst geringer Umrüstarbeit für beide Systeme verwendbar ist. Dabei soll diese Umrüstung auch vom verwendenden Zahnarzt durchgeführt werden können, wenn schon nicht während der Behandlung eines Patienten, so doch bei der Entscheidung, welche Winkelstücke er mit welchen Handstücken verwenden will.

Erfindungsgemäß ist dabei vorgesehen, daß der Schleifring, der im Falle des die Lichtquelle enthaltenden Winkelstückes diese trägt, gegen einen Ring ausgetauscht werden kann, der an Stelle der Lampe einen durchgehenden Lichtleiter aufweist und daß bei diesem Ring an der Stelle, an der die Winkelstücke ohne Lampe eine Nase aufweisen, ebenfalls eine Nase vorgesehen ist.

Diese Lösung ist deshalb möglich, weil die Schleifringe bei den Winkelstücken mit Lichtquelle im Winkelstück zum Zwecke des Lampenaustausches die Schleifringe vom Zahnarzt selbst leicht aus dem Winkelstück entfernbar und ebenso leicht in dieses wieder einsetzbar sein müssen. Es ist daher für den Zahnarzt der erfindungsgemäß ermöglichte Wechsel des Kupplungssystemes ebenso leicht durchzuführen wie bisher der Wechsel der Lampe.

Für die Hersteller der Hand- und Winkelstücke bringt die Erfindung eine wesentliche Reduktion der Lagerhaltungs-, Arbeitsvorbereitungs-, und Auslieferungskosten.

Die Erfindung wird an Hand der Zeichnungen näher erläutert. Dabei zeigt:
Fig. 1 ein zahnärztliches Winkelstück und ein die Lichtquelle aufweisendes Handstück in der Lage unmittelbar vor oder nach dem ein- bzw. auskuppeln;
Fig. 2 ein zahnärztliches Winkelstück mit Lichtquelle und ein dazugehöriges Handstück in einer Lage analog zu der in Fig. 1;
Fig. 3 und 4 stellen analoge Darstellungen zu den Figura 1 und 2 in perspektivischer Ansicht dar;
Fig. 5 zeigt das Austauschen der Lichtquelle, wenn diese im Winkelstück untergebracht ist und
Fig. 6 zeigt den erfindungsgemäßen Kupplungsring, den die Lichtquelle tragenden Schleifring und ihre Lage bezüglich des Winkelstückes.

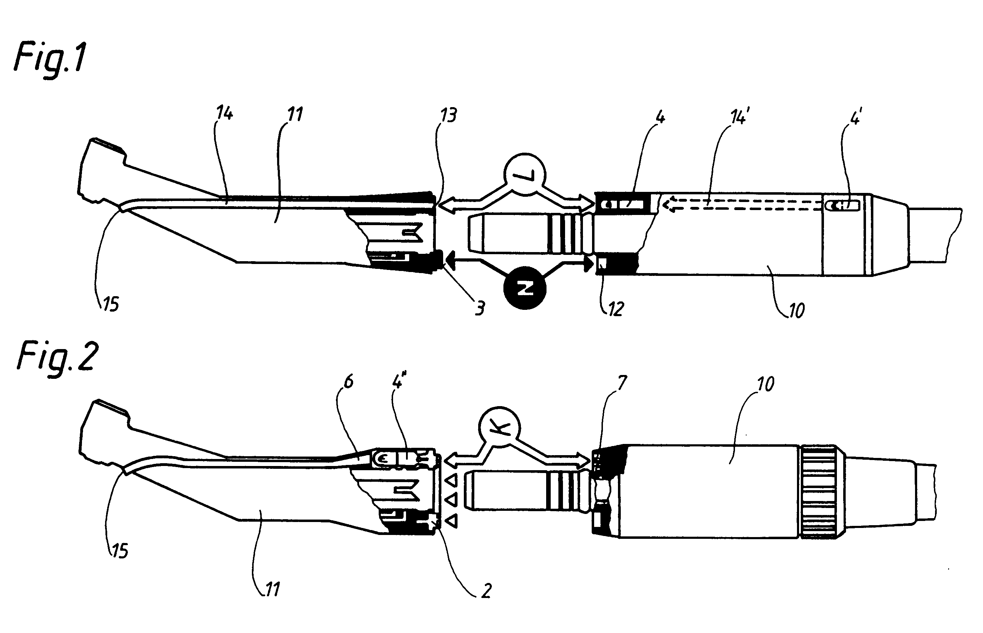

Eine übliche Handstück 10 - Winkelstück 11 - Kombination, bei der die Lichtquelle 4, 4' im Handstück untergebracht ist, ist in Fig. 1 dargestellt. Die Lage der beiden Teile zueinander entspricht der Situation unmittelbar vor dem Verbinden bzw. unmittelbar nach dem Lösen der Verbindung. Die Lichtquelle 4, 4' kann dabei direkt im Kupplungsbereich oder am apparateseitigen Ende des Handstückes 10 untergebracht sein, wobei in letzterem Fall ein Lichtleiter 14' das Licht von der Quelle 4' zum Kupplungsbereich 4 leitet.

Eine Nase N im Kupplungsbereich des Winkelstückes 11 wirkt mit einer Vertiefung 12 im Handstück 10 so zusammen, daß das handstückseitige Ende 13 des im Winkelstück 11 befindlichen Lichtleiters 14 der Lichtquelle 4 bzw. gegebenenfalls dem winkelstückseitigen Ende eines Lichtleiters 14' gegenüberliegt, um das Licht zum werkzeugseitigen Ende 15 des Lichtleiters 14 und damit auf die Arbeitsstelle zu lenken.

Fig. 2 zeigt die zweite weitverbreitete Bauart, bei der eine Lichtquelle 4'' im Winkelstück 11 vorgesehen ist. Diese Lichtquelle ist auf einem demontierbaren Schleifring 2 angebracht und steht dem kupplungsseitigen Ende eines Lichtleiters 6 gegenüber. Das Licht tritt wiederum bei 15 aus dem Lichtleiter 6, um das Operationsgebiet zu beleuchten. Die Stromversorgung für die Lichtquelle 4'' erfolgt über stirnseitig angebrachte konzentrische Leiter im Schleifring 2 und entsprechende Stifte im Handstück 10.

Die Kupplungen beider Systeme entsprechen einander mit Ausnahme der geschilderten Beleuchtungseinrichtung. Insbesondere die Kraftübertragung, die Übertragung der Kühl- und Spülflüssigkeiten etc. ist bei beiden Arten von Kupplungen identisch.

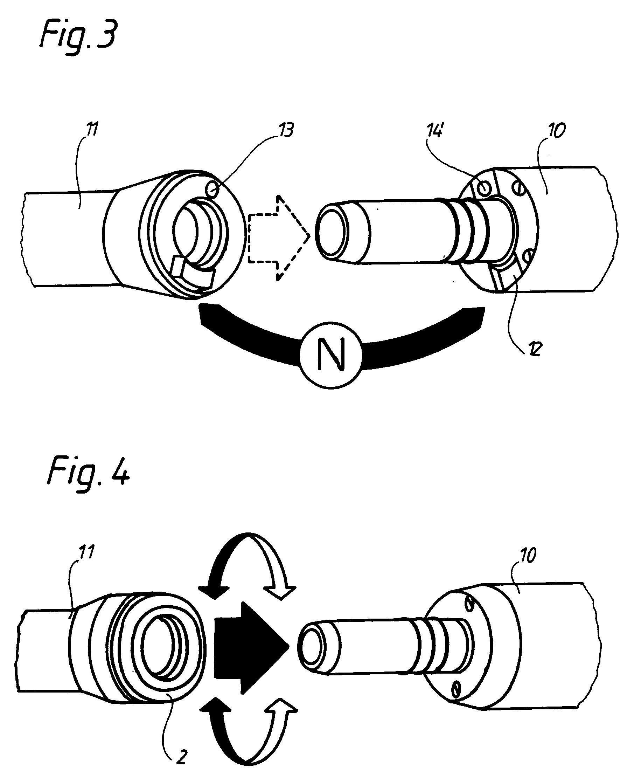

Die Fig. 3 und 4 zeigen die Situation gemäß Fig. 1 und 2 in perspektivischer Ansicht, wobei in Fig. 3 ein Handstück 10 dargestellt ist, das über einen Lichtleiter 14' verfügt. In Fig. 4 ist die stirnseitige Ausbildung der Leiterbahnen am Schleifring 2 gut zu erkennen.

Man erkennt weiters die freie Drehbarkeit zwischen dem Winkelstück und dem Handstück im Falle der Fig. 4 und die drehfeste Verriegelung bei der Ausführungsform gemäß Fig. 3.

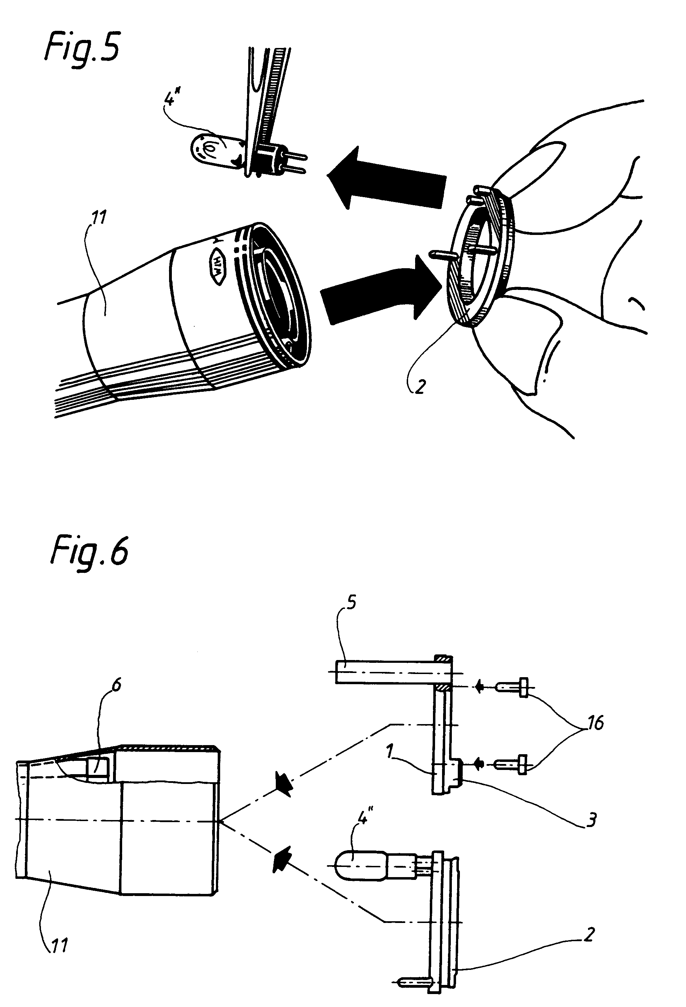

Fig. 5 zeigt das Auswechseln einer durchgebrannten, im Winkelstück untergebrachten Lampe, die auf dem Schleifring 2 des Winkelstückes 11 angebracht ist. Diese leichte Ausbaubarkeit des Schleifringes 2 macht sich die Erfindung zu Nutze, indem sie, wie in Fig. 6 dargestellt, einen Kupplungsring 1 verwendet, der über eine Nase 3 in dem Bereich verfügt, indem die Norm für Lichtquellen im Handstück sie vorsieht und bei dem anstelle einer Lampe 4'' ein Stück eines Lichtleiters 5 angebracht ist, der das Licht von der Lichtquelle 4 bzw. dem Lichtleiter 14' im Handstück zum Lichtleiter 14 im Winkelstück weiterleitet.

Gegebenenfalls kann durch Schrauben 16, die in Fig. 6 winkelverdreht eingezeichnet sind, eine festere, aber nach wie vor lösbare Verbindung zwischen dem Kupplungring 1 und dem Winkelstück 11 vorgesehen sein.

Wie aus der nebeneinander gezeichneten Darstellung der beiden Ringe 1, 2 ersichtlich, ist es auf Grund der Erfindung möglich, einheitliche Winkelstücke 11 herzustellen und als Zubehör die beiden Ringe 1, 2 mitzuliefern oder im Werk alternativ zu montieren, wodurch beide Arten handstückseitiger Kupplungen verwendet werden können. Dies bringt für den Produzenten eine wesentliche Erleichterung bei der Herstellung und Lagerhaltung und für den Verwender die Möglichkeit frei die Handstücksysteme wechseln zu können, ohne auch die gesamten Winkelstücke neu beschaffen zu müssen.

## Patentansprüche

1. Kupplung, um zahnärztliche Winkelstücke mit Handstücken zu verbinden, wobei im Winkelstück nahe des Werkzeughalters eine Austrittsstelle für einen Lichtleiter vorgesehen ist, um die Arbeitsstelle im Mund des Patienten zu beleuchten und wobei die entsprechende Lichtquelle entweder im Winkelstück oder im Handstück vorgesehen ist, wobei bei winkelstückseitiger Lampe diese auf einem kupplungsseitigen Schleifring sitzt, dadurch gekennzeichnet, daß der Schleifring (2), der im Falle des die Lichtquelle (4'') enthaltenden Winkelstückes (11) diese trägt, gegen einen Ring (1) ausgetauscht werden kann, der an Stelle der Lampe (4'') einen durchgehenden Lichtleiter (5) aufweist und daß bei diesem Ring (1) an der Stelle, an der die Winkelstücke (11) ohne Lampe (4'') eine Nase (N) aufweisen, ebenfalls eine Nase (N) vorgesehen ist.

2. Kupplung nach Anspruch 1, dadurch gekennzeichnet, daß der Kupplungsring (1) mittels Schrauben (16) am Winkelstück befestigt ist.

## Claims

1. A coupling for connecting dental elbows with handpieces, wherein an outlet point for a light conductor is provided in the elbow near to the tool holder so as to illuminate the work location in the patient's mouth and wherein the corresponding light source is provided either in the elbow or in the handpiece, wherein if the light is provided in the elbow it is mounted on a slip ring on the coupling side thereof, characterised in that the slip ring (2), which in the case of the elbow (11) containing the light source (4'') carries the latter, can be exchanged for a ring (1) which at the location of the light (4'') has a continuous light conductor (5), and in that a lug (N) is also provided in this ring (1) at the location at which the elbows (11) without a light (4'') have a lug (N).

2. A coupling according to Claim 1, characterised in that the coupling ring (1) is secured to the elbow by means of screws (16).

## Revendications

1. Accouplement pour relier des pièces coudées dentaires et des pièces à main, dans lequel il est prévu dans la pièce coudée à proximité du porte-outils un point de sortie pour un guide de lumière pour éclairer le site de travail dans la bouche du patient, la source lumineuse correspondante étant prévue dans la pièce coudée ou dans la pièce à main, dans lequel, lorsque la lampe est du côté de la pièce coudée, elle repose sur une bague glissante située du côté de l'accouplement, caractérisé en ce que la bague glissante (2), qui porte la source lumineuse (4'') lorsque la pièce coudée (11) contient celle-ci, peut être remplacée par une bague (1) qui comporte, à la place de la lampe (4''), un guide de lumière traversant (5), et en ce que, sur cette bague (1), à l'endroit où les pièces coudées (11) sans lampe (4'') comportent un tenon (N), il est prévu de même un tenon (N).

2. Accouplement selon la revendication 1, caractérisé en ce que la bague d'accouplement (1) est fixée sur la pièce coudée à l'aide de vis (16).
